**Europäisches Patentamt**

**European Patent Office**

**Office européen des brevets**

(19)

(11) Publication number: **0 366 480 B1**

## EUROPEAN PATENT SPECIFICATION

(45) Date of publication of patent specification: **24.08.94**    (51) Int. Cl.⁵: **A61K 31/20**

(21) Application number: **89311089.0**

(22) Date of filing: **27.10.89**

The file contains technical information submitted after the application was filed and not included in this specification

(54) **Method and compositions for treating Alzheimer's disease, related dementias & epilepsy.**

(30) Priority: **27.10.88 US 263540**
**01.06.89 US 359562**
**06.07.89 US 376289**
**27.09.89 IL 91802**

(43) Date of publication of application:
**02.05.90 Bulletin 90/18**

(45) Publication of the grant of the patent:
**24.08.94 Bulletin 94/34**

(84) Designated Contracting States:
**AT BE CH DE ES FR GB GR IT LI LU NL SE**

(56) References cited:
**EP-A- 0 234 733**
**EP-A- 0 275 643**

(73) Proprietor: **BAR ILAN UNIVERSITY**
**P.O. Box 1530**
**IL-52100 Ramat-Gan (IL)**

(72) Inventor: **Yehuda, Shlomo**
**17 Beeri Street**
**Tel Aviv (IR)**

(74) Representative: **Collier, Jeremy Austin Grey et al**
**J.A.KEMP & CO.**
**14, South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

FIELD AND BACKGROUND OF THE INVENTION

The present invention relates to compositions containing two particular unsaturated fatty acids, for treating Alzheimer's disease, related dementias and epilepsy.

In European published Patent Application No. 0275643 (and in corresponding patent applications in other countries) there are disclosed inter alia a composition of matter of linolenic and linoleic acids (including physiologically hydrolyzable and pharmacologically acceptable derivatives thereof) within a specified percentage range, a pharmaceutical formulation and a nutritional composition each containing linolenic and linoleic acids within the same percentage range, and a method for treating a mammal for the purpose of inducing therein at least one physiological effect selected from memory enhancement, analgesia, sleep regulation and inhibition of the symptoms of senility.

In the aforesaid applications, the disclosures of which are incorporated herein by reference, details of experiments were recorded, in which it was shown that a mixture of the above carboxylic acids within the specified percentage range effected an improvement in memory, an increase of the pain threshold and a sleep regulation effect in healthy rats. A similar mixture of the two acids also appeared to indicate a memory improvement in rats which had a memory deficit due to aging or to iron deficiency. Moreover, there appeared to be indications that a number of geriatric patients improved in certain respects after administration of the mixture of acids. From the foregoing, it will be apparent that there was no evidence that symptoms of any particular diseases were susceptible to alleviation by administration of the acid mixture.

EP-A-234733 proposes the treatment of pre-senile or senile dementia, including Alzheimer's disease, with a lithium compound or an essential fatty acid, a term stated to refer to linoleic acid, gamma-linolenic acid, dihomogammalinolenic acid, arachidonic acid, adrenic acid, alpha-linolenic acid, eicosapentaenoic acid, and docosahexaenoic acid.

Senile dementia of Alzheimer's type (more commonly known as "Alzheimer's disease") appears to be the most widespread neuropsychiatric disease and is becoming increasingly prevalent with the general aging of the population. It is not, however, confined to the elderly, as this term is generally understood. To the best of the inventor's knowledge, no satisfactory treatment for this type of dementia or for related dementias has yet been evolved.

It is an object of the present invention to provide a medicament for treatment for Alzheimer's disease and related dementias, which relieves at least certain symptoms thereof.

The term "epilepsy" denotes a group of central nervous system disorders characterized by transitory episodes of abnormal motor, sensory, autonomic or psychic phenomena, and are almost always correlated with abnormal and excessive discharges in the encephalogram. Most known useful antiepileptic agents are either barbiturates or are structurally related thereto, or are benzodiazepines, dibenzoazepines, or lipids or lipid-like compounds containing phosphorus and/or nitrogen. Valproic acid ($Pr_2CHCOOH$) and its derivatives are also useful in this respect. However, to the inventor's knowledge, it has not hitherto been proposed to use polyunsaturated unbranched long-chain carboxylic acids, containing only C, H and O, as antiepileptic compounds.

It is a further object of the present invention to provide a medicament for treatment for epilepsy, which relieves at least certain symptoms thereof.

The present invention provides the use for the manufacture of a medicament for therapeutic application in treatment of Alzheimer's disease, related dementias and epilepsy of a composition which comprises a combination of (a) from 16.7 to 22.2% by weight of at least one compound selected from linolenic acid and derivatives thereof, calculated as the free acid, said derivatives of linolenic acid being both physiologically hydrolysable and pharmacologically acceptable, and (b) balance to make 100% of said combination, of at least one compound selected from linoleic acid and derivatives thereof, calculated as the free acid, said derivatives of linoleic acid being both physiologically hydrolysable and pharmacologically acceptable, provided that said composition does not include oily carrier or diluent selected from $C_{8-18}$ saturated fatty acids, oleic acid and derivatives of these acids.

A pharmaceutical formulation which comprises ingredients (a) and (b) as identified in, and in the proportions recited in, the foregoing paragraph, may also include at least one pharmaceutically acceptable substance selected from diluents, carriers and adjuvants, except an oily carrier or diluent which comprises at least one member of the group consisting of $C_{8-18}$ saturated fatty acids, oleic acid and derivatives of these acids.

Alternatively, there may be utilized a nutritional composition, adapted for consumption by humans, comprising an orally ingestible diluent or carrier, except an oily carrier or diluent which comprises at least one member of the group consisting of $C_{8-18}$ saturated fatty acids, oleic acid and derivatives of these acids, and ingredients (a) and (b) as identified above in the stated proportions.

For purposes of definition, it should be noted that such terms as "method for the treatment of epilepsy", "alleviation of symptoms", and similar expressions, where used in the present specification and claims, are intended to include inter alia any such treatment which is effective to reduce the incidence and/or intensity of epileptic occurrences in subjects susceptible to such occurrences. Moreover, it should be noted that references herein to a method of treating Alzheimer's disease, related dementias and epilepsy, encompass prophylactic methods, including cases in which symptoms of disease are absent.

In accordance with the invention, it has been found to be advantageous to utilize, for treating Alzheimer's disease, related dementias and epilepsy, a composition of matter which consists of from 16.7 to 22.2% by weight of component (a) and 83.3 to 77.8% by weight of component (b), and more particularly of a composition of matter which consists of about 19.0% by weight of component (a) and about 81.0% by weight of component (b).

These preferred percentage proportions by weight are also applicable to the relationship between the at least one compound selected from linolenic acid and physiologically non-deleterious and hydrolyzable derivatives thereof, and the at least one compound selected from linoleic acid and physiologically non-deleterious and hydrolyzable derivatives thereof (calculated as the free acids), in the nutritional compositions of the invention.

Since, as has been intimated above, it is believed that the combination of linoleic and linolenic acids is the active principle per se which is effective in the method for treating Alzheimer's disease, related dementias and epilepsy, it will be appreciated by those skilled in the art that instead of the acids themselves, there may be utilized in the composition of the invention derivatives of these acids which are both physiologically hydrolyzable (to the corresponding acids) and pharmacologically acceptable. Such derivatives may for example be selected from salts, esters and amides of the respective acids.

Among suitable salts there may be mentioned the ammonium, sodium, potassium, calcium and magnesium salts as salts with substituted mono- and di-substituted amines and the analogous saturated heterocyclic compounds containing an NH group in the ring, so long as the amines and the analogues in question are physiologically acceptable. As suitable esters there may be mentioned, for example, the ethyl and glyceryl esters. Amides of the acids may also be utilized, e.g. amides of the acids with substituted mono- and di-substituted amines and with the analogous saturated heterocyclic compounds containing an NH group in the ring, so long as the amines and the analogues in question are physiologically acceptable. It will be appreciated that the latter stipulation is necessary (in the case of the amine salts, the amides and their heterocyclic analogues) since it is to be expected that such derivatives will metabolize in the body to the desired acids and the starting amines or heterocyclic compounds. It will of course be evident to a person skilled in the art how to select a particular salt, ester or amide, so as to comply with the requirements of physiologically hydrolyzing to the corresponding acids, and pharmacological acceptability.

The pharmaceutical formulation which is utilizable in accordance with the present invention may be adapted for oral, parenteral or rectal administration, and it may be in the form of dosage units. The diluents, carriers and adjuvants are those conventionally used in pharmaceutical and veterinary formulation.

For oral administration, the pharmaceutical formulations may be utilized as e.g. tablets, capsules, emulsions, solutions, syrups or suspensions. For parenteral administration, the formulations may be utilized as ampoules, or otherwise as suspensions, solutions or emulsions in aqueous or oily vehicles. The need for suspending, stabilizing and/or dispersing agents will of course take account of the fact of the solubility or otherwise of the linoleic and linolenic acids, or of their derivatives used in the formulations, in the vehicles which are used in particular embodiments. Thus, for example, where the acids themselves are used, account will be taken of the fact that these have a relatively low water solubility and in general a relatively high oil solubility. The formulations may additionally contain e.g. physiologically compatible preservatives and antioxidants.

The pharmaceutical formulations may also be utilized as suppositories with conventional suppository bases such as cocoa butter or other glycerides. As is well known in the pharmaceutical art, the formulations may also be made available in a depot form which will release ingredients (a) and (b) slowly in the body, over a preselected time period.

The nutritional composition which may be utilized according to the invention includes as a necessary component an orally ingestible diluent or carrier (subject to the exception noted herein); this may for example comprise a substance selected from sugar-based confectionery, a manufactured cereal, a fruit or vegetable product, a beverage or beverage concentrate, or any inert diluent, carrier or excipient known in the pharmaceutical art. It is intended generally that ingredients (ii) and (iii), as previously defined, may be used in nutritional compositions in any of the forms in which these are known and practised in the art. Thus, the nutritional compositions may take the form of, e.g., sugar-based confectionery such as candies or chocolate, breakfast cereals, fruit or vegetable purees or beverages, other beverages (including those based on carbonated water), or beverage concentrates generally (including those in the form of e.g. powders, granules, flakes or crystals, which are intended to be mixed with hot or cold water and/or milk). The nutritional compositions may also generally be in the form of powders, tablets, capsules, solutions, concentrates, syrups, suspensions, gels or dispersions. It will be evident that when the nutritional compositions take the form of dispersions or suspensions, it will usually be necessary to use an acceptable (i.e. non-toxic and otherwise suitable) dispersing or suspending agent, as is well known in the nutritional and pharmaceutical arts. When these compositions are utilized in the form of capsules, it will be evident that gelatin or other known suitable ingestible materials may be used for encapsulation.

The present invention further includes the utilization of nutritional compositions which also include any of the known vitamins. Thus for example, such compositions (which may be, but need not be, in the form of aqueous suspensions) may comprise at least one water-soluble vitamin selected from thiamine, riboflavin, niacin, pyridoxine, pantothenic acid, biotin, folic acid, cobalamin and ascorbic acid. Alternatively or additionally, such compositions may comprise at least one oil-soluble vitamin selected from retinol, calciferol, tocopherol and menadione. The nutritional compositions of the present invention may also comprise in combined form at least one element selected from sodium, potassium, calcium, phosphorus, magnesium, chlorine and sulfur, and additionally or alternatively, at least one element selected from iron, copper, iodine, manganese, cobalt, zinc, molybdenum, fluorine, selenium and chromium. These compositions may also contain other natural or synthetic antioxidants.

The nutritional compositions may also comprise other unsaturated fatty acids, such as for example those known to be metabolized in the body to prostaglandins, e.g. dihomo-gamma-linolenic acid, arachidonic and eicosapentaenoic acids, as well as physiologically compatible derivatives thereof, such as salts, esters and amides of such acids.

The present invention will now be illustrated by the non-limitative Examples which follow.

EXAMPLE I: TESTS ON ALZHEIMER'S DISEASE PATIENTS

Selection of Patients

Alzheimer's disease was defined according to the Diagnostic & Statistical Manual of the American Psychiatrists Association, 3rd Edn., March 1980. The criteria for inclusion are: complaints of disorientation in space and time, cognitive deficits and low scores in the Mini-Mental Test. Criteria for exclusion from the study are: multi infarction dementia, depressive or post-depressive dementia, post-traumatic dementia, post-psychotic dementia, known endocrine disorder, normintensive hydrocephalus, very aggressive patient, very severe condition requiring constant assistance in the daily routine.

Criteria for Assessment of Results

The guardian was asked to assess the severity of the patient's condition in 12 areas of behaviour on a five-point scale, a score of 5 indicating a severe problem, a score of 1 indicating no problem. The 12 areas were as follows:

1. Space orientation: can the patient find the way to return alone without confusion and without losing sense of direction?
2. Level of cooperation with family and doctors etc.
3. General mood of patient, especially whether aggressive.
4. The patient's appetite, attention to food, time of meals, interest in food.
5. Ability of patient to keep belongings in order and organize his life.
6. Short-term memory - ability to remember recent events.
7. Long-term memory - ability to remember remote events.
8. Sleep habits and sleep disturbances.
9. Whether patient is alert during daytime: periods of alertness and span of attention.
10. Does patient have auditory or visual hallucinations; if so, at what time of day?
11. Is patient capable of self-expression in clear speech and ideas?
12. Control of urination.

Method

Treatment in accordance with the present invention (or placebo) were carried out on 100 patients (79 male and 21 female; 24 of the 100 were hospitalized), of ages in the range 50-73 years. 71 of the patients had been definitely diagnosed as having Alzheimer's disease at least 4 years previously; in the past they had been treated with hydergin, various cholinergic drugs, piracetam or/and lecithin. The present treatment was administered to 60 patients, while 40 received placebo. The following table summarizes the patient sample.

| Classification of Alzheimer's Patients | | | | |
|---|---|---|---|---|
| | Hospitalized | | Non-hospitalized | |
| | M | F | M | F |
| Treatment | 9 | 3 | 40 | 8 |
| Placebo | 9 | 3 | 21 | 7 |
| Totals | 18 | 6 | 61 | 15 |

Patients received oral doses, 1 ml. morning and evening, of either medication or placebo, over a period of three weeks; the medication, which was kept in the cold or refrigerated, comprised per ml. 0.25 ml. of a mixture of linolenic and linoleic acids (Sigma) in a 1:4.25 weight ratio (19.05%: 80.95%), 0.73 ml. paraffin oil, 0.02 ml. alphatocopherol (Vitamin E, Sigma) and a few drops of flavoring (almond essence). At the end of the three-week period, each patient was medically reexamined, EEG recorded and samples of blood and urine were tested. A Mini Mental test was administered and the guardian was again asked to rate the severity of complaints on the basis of the above 12 questions.

Results

(1) The following table summarizes scores in the Mini Mental Test. The stated figures are average values for the number of patients (n).

| Treatment of Alzheimer's Patients - Mini Mental Test Scores | | | |
|---|---|---|---|
| | Placebo(n = 40) | Treatment(n = 60) | |
| | | responders (n = 49) | non-responders (n = 11) |
| Before | 7.3 ± 3.1 | 7.8 ± 2.9 | 7.6 ± 3.2 |
| After | 7.5 ± 3.5 | 16.0 ± 3.7* | 8.0 ± 2.5 |

*P<0.01

(2) The following table summarizes the results of assessment of patients' condition in the 12 areas detailed above, on the 5-point scale, before and after treatment. A change of at least 1.4 units is considered significant. Each fraction denotes:

$$\frac{\text{number of patients improved}}{\text{number of patients with severe problem}}.$$

| Treatment of Alzheimer's Patients - Ratings in 12 Areas | | | | |
|---|---|---|---|---|
| AREA | Placebo (n = 40) | | Treatment (n = 60) | |
| | fraction improved | % | fraction improved | % |
| 1. Space orientation | 3/33 | 9.0 | 37/50 | 74.0 |
| 2. Cooperativeness | 2/31 | 5.1 | 28/49 | 57.1 |
| 3. Mood | 5/27 | 18.5 | 27/44 | 61.4 |
| 4. Appetite | 2/31 | 5.1 | 26/48 | 54.2 |
| 5. Organization | 4/32 | 12.5 | 33/48 | 68.7 |
| 6. Short-term memory | 1/34 | 2.9 | 40/59 | 74.0 |
| 7. Long-term memory | 0/38 | 0 | 34/58 | 58.7 |
| 8. Sleep problems | -2/27 | -7.4 | 21/29 | 74.4 |
| 9. Daytime alertness | -2/33 | -5.1 | 29/47 | 61.7 |
| 10. Hallucinations | -2/10 | -20.0 | 12/14 | 85.0 |
| 11. Self-expression | 1/36 | 2.7 | 16/52 | 30.7 |
| 12. Bladder control | 3/14 | 21.4 | -2/27 | -7.4 |

(3) General physiological effects.

No major side effects were found after the three week treatment period; one patient only suffered from severe stomach upset and diarrhea. Body temperature and blood pressure (systolic and diastolic) were unchanged at the end of this period. Biochemical blood and urine laboratory tests did not show significant changes after treatment. There was no increase in total lipids in the blood. There was a tendency of the blood cholesterol level to decrease, but this was not necessarily of statistical significance.

EXAMPLE II: FURTHER TESTS ON ALZHEIMER'S DISEASE PATIENTS

A further group of 13 Alzheimer's Disease out-patients, 9 male and 4 female, in the age range of 59-71 years, was treated with the same oral medication (no placebo), in a similar manner to Example 1.

Results

(1) The following table summarizes scores in the Mini Mental Test. The stated figures are average values for the number of patients (n).

| Treatment of Alzheimer's Patients - Mini Mental Test Scores | |
|---|---|
| | Treatment(n = 13) |
| | responders (n = 10) |
| Before | 7.0 ± 1.7 |
| After | 15.6 ± 2.1* |

*P<0.01

(2) The following table summarizes the results of assessment of patients' condition in the 12 areas detailed above, on the 5-point scale, before and after treatment. A change of at least 1.4 units is considered significant. Each fraction denotes:

$$\frac{number\ of\ patients\ improved}{number\ of\ patients\ with\ severe\ problem}.$$

| Treatment of Alzheimer's Patients - Ratings in 12 Areas | | |
|---|---|---|
| AREA | Treatment (n = 13) | |
| | fraction improved | % |
| 1. Space orientation | 8/12 | 66 |
| 2. Cooperativeness | 5/12 | 42 |
| 3. Mood | 5/ 9 | 55 |
| 4. Appetite | 6/11 | 55 |
| 5. Organization | 8/11 | 72 |
| 6. Short-term memory | 10/12 | 83 |
| 7. Long-term memory | 9/12 | 75 |
| 8. Sleep problems | 4/ 6 | 66 |
| 9. Daytime alertness | 5/11 | 45 |
| 10. Hallucinations | 5/ 6 | 83 |
| 11. Self-expression | 5/12 | 42 |
| 12. Bladder control | 0/ 7 | 0 |

Unwanted side effects were not observed in the group of 13 patients. It is seen that the results in Example II are in line with the noted improvement in the condition of Alzheimer's patients found in Example 1, when the method according to the present invention is utilized.

EXAMPLE III: TESTS ON ANIMAL MODELS OF EPILEPSY

Preliminary note.

Tests on animal models enable possible antiepileptic drugs to be evaluated for potential therapeutic use in humans. Such tests measure the protection afforded by the drug against the convulsant effects of chemical or electrical stimulants. An important chemical stimulant utilized for these tests is pentylenetetrazol (PTZ, Metrazol-Knoll), which has been used in experimental animals to induce grand mal seizures.

Example IIIa

The inventive composition (labelled "SR-3") administered to animal models in accordance with the method of the invention comprised per ml. 0.40 ml. of a mixture of linolenic and linoleic acids (Sigma) in a 1:4 weight ratio (20% : 80%), 0.59 ml. paraffin oil, and 0.01 ml. alpha-tocopherol (Vitamin E, Sigma).

In a first stage, the $ED_{50}$ for PTZ-induced grand mal seizures in SPD rats was investigated and found to be 76.5 mg./kg. The $LD_{50}$ is tightly closed at 81.0 mg./kg.

In a second stage, 80 (150 g.) male rats were divided into two groups. One group received 0.2 ml. i.p. saline treatment daily (40 rats) and the other group received treatment with 0.2 ml. i.p. SR-3 daily (40 rats), for a period of three weeks. After the three-week treatment, the rats were given one of two doses of PTZ, 50 mg./kg. or 100 mg./kg. The rats were observed by two independent observers, who were ignorant of which treatment each rat received. An EEG recording was not performed. The following variables were recorded in Table 7, below:

1. latency (seconds) to the first grand mal seizure (tonic-clonic contractions of the limbs, trunk and head, falling, saliva and blood discharge from the mouth);
2. number of rats responding with grand mal seizures;
3. mean duration (in seconds) of the grand mal seizures;
4. number of rats that died not exhibiting grand mal seizures but showing "infantile spasms" (sudden flexion of the forelegs, forward flexion of the trunk, or extension of the rear legs), the attack lasting only a few seconds, but it was repeated several times;
5. number of rats that died 15 minutes after PTZ injection.

7

Table 7

| Study of the effect of SR-3 on animal models of PTZ-induced epilepsy | | | | | | |
|---|---|---|---|---|---|---|
| Pretreatment | PTZ dose (mg./kg.) | Variable studied | | | | |
| | | 1 | 2 | 3 | 4 | 5 |
| Saline* | 50 | 28±12 | 8 | 637±31 | 8 | 9 |
| SR-3* | 50 | 252±11 | 1 | 24 | 1 | 0 |
| Saline* | 100 | 7± 3 | 19 | 893±14 | 1 | 20 |
| SR-3* | 100 | 154±25 | 3 | 27± 6 | 4 | 2 |

*number of rats in each group = 20

The results clearly showed that pretreatment in accordance with the invention protected the rats from grand mal seizures induced by PTZ. Most of the rats (19/20) receiving the placebo that were challenged with 100 mg./kg. PTZ exhibited clear cut grand mal seizures. It is of interest to note that the seizure began shortly after the PTZ injection, and lasted for about 15 minutes without interruption, until death occurred. All of these rats were dead 15 minutes after the PTZ injection. In contrast, rats pretreated with SR-3 and later challenged with 100 mg./kg. PTZ were very much protected. Only 3 out of 20 of this group showed grand mal seizures, and even in these 3 cases the attacks lasted only about 25 seconds; only 2 of the rats exhibiting seizures were dead 15 minutes after the PTZ injection. Another 12 rats from this group were found dead 4 hours after the PTZ injection.

Similar SR-3 protection was found in the group receiving the smaller dose. In the rats receiving saline solution, 8 out of the 20 had grand mal seizures with a latency of about 0.5 minute. The duration of the attacks was shorter than in the group receiving the larger PTZ dose. Only one rat from the SR-3 group showed a brief grand mal attack. In the group receiving the placebo, 9 rats were dead 15 minutes after the PTZ injection. No immediate deaths occurred in the SR-3 group and only 3 rats in this group were found dead 4 hours later; all others recovered.

Example IIIb

The use of ferrous or ferric salts injected into the cortex or amygdala-hippocampus complex for the induction of epileptogenic discharge (epileptic foci) is well-known. A group of 50 SPD male rats were prepared for this experiment by implantation of canules in their brain. The tip of the canules was in the amygdala according to Czernansky et al (Life Sciences, 1988, 32: 385-390). Then, 25 rats received SR-3 in daily injections as in Example IIIa. The other 25 rats received a daily injection of saline (0.9% NaCl). After 3 weeks of treatment, all rats received 100 uM $FeCl_3$ intraventricularly through the canules into the amygdala. Only 2 out of 25 rats which were treated with saline did not exhibit epileptic seizure, while 21 of the 25 rats pretreated with SR-3 were protected from $FeCl_3$-induced epileptic seizures.

Example IIIc

In this method of investigation according to Craig, C.R. and Colasanti, B.J., 1989 Pharmacol. Biochem. Behav. 31: 867-70, PTZ was used to induce seizure and the ability of SR-3 to protect from seizure was examined. 15.0 mg./kg. was injected every 15 minutes until seizure occurred. For this purpose, seizure is defined as a single episode of colonic spasms of both forelimbs and hindlimbs lasting at least 5 secs., followed by loss of righting reflex. The results in the following table are expressed as the number of periods to seizure (mean + S.D.; ANOVA = P<.001).

| | First Week | Second Week | Third Week |
|---|---|---|---|
| Control (n = 25) | 4.32 ± 1.30 | 3.72 ± 1.41 | 3.50 ± 1.80 |
| SR-3 (n = 25) | 18.64 ± 3.09 | 19.94 ± 3.21 | 22.75 ± 3.45 |
| p< | 0.001 | 0.001 | 0.001 |

The above results show that SR-3 afforded clear protection, insofar as many more injections of PTZ were needed to evoke seizure in the SR-3 group than in the control group, with no apparent habituation from one week to another.

Example IIId

In this model, 55 non-audiogenic rats were made audiogenic (i.e. they are responsive with seizure to auditory stimuli) by chronic administration of p-cresol over a 7-week period; the effect of p-cresol lasts 4-5 weeks (see Yehuda, S. et al, 1977 Internat. J. Neurosci. 7: 223-6). Then, 27 rats were treated with saline and 28 rats received SR-3, after which all rats were subjected to the same auditory stimuli and observed for 4 hours. Seizures were observed in 23 out of the 27 control rats, but in only 6 out of the 28 SR-3 treated rats.

The overall results in Example III indicates that the composition of the invention affords protection from seizures even when the animals under investigation were already seizure-prone.

EXAMPLE IV: THE EFFECT OF OTHER FATTY ACIDS & NATURAL OILS ON LEARNING, MEMORY AND EPILEPSY

Preliminary Note: Reference in this Example to learning and memory is intended to refer mainly to experiments analogous to those recorded in my prior patent applications mentioned above. Moreover, while the present invention is not to be limited by any theory, nevertheless, it is believed that the experiments in this Example tending to show the negative effects of natural oils and fatty acids other than those specified in the present inventive combination, on memory and learning, may imply also a similar negative effect as regards the symptom-alleviating properties of such combination on Alzheimer's disease.

Example IVa

The following natural oils were administered to rats (c.f. Example 1 of my prior patent applications):

|  | linoleic acid(%) | linolenic acid(%) |
|---|---|---|
| corn oil | 53.3 | 1.0 |
| linseed oil | 14.9 | 53.0 |
| olive oil (European) | 8.7 | 1.0 |
| palm oil | 10.2 | 0.5 |
| sunflower oil | 75.5 | 0.8 |
| soybean oil | 52.4 | 8.9 |

None of these oils had any beneficial effects on the learning capacity of normal rats, or improved the learning deficit induced by iron-deficient diet. Moreover, none of these oils gave any protection from PTZ-induced seizures. Also, mixtures of linseed oils with other oils in proportions such that the linolenic acid: linoleic acid ratio was similar to that of SR-3 (above), had no effect on learning or epilepsy. The amount of oil given to each rat was similar to the SR-3 dosage mentioned above.

Example IVb

When linolenic acid was added to corn oil, olive oil or sunflower oil, or when linoleic acid was added to linseed oil, in order that the linolenic:linoleic acid ratio therein (calculated as free acids) should be 1:4 by weight, such compositions had no effect on learning or epilepsy. Also, when a mixture of free linoleic and linolenic acids in this ratio was mixed with free palmic acid or free stearic acid, as carrier, such compositions had no effect on learning or epilepsy, while substitution of oleic acid for the palmic or stearic acid in these experiments gave inconclusive results.

It is concluded that palmic, stearic and (probably) $C_{8-18}$ saturated fatty acids generally and oleic acid, as well as derivatives thereof, such as those occurring in natural oils, when present in carriers or diluent quantities, adversely affect the beneficial properties of the inventive compositions, and also consequently that natural oils are unsuitable for use as carriers or diluents for the compositions of the invention. A person skilled in the art will be able to determine without undue experimentation whether or not smaller than carrier quantities of the thus excluded acids (and their derivatives), would adversely affect the advantageous biological activity of the inventive combination of linolenic and linoleic acids. Insofar as it may be found that these smaller quantities would not unduly affect such activity, compositions including them besides the

9

inventive combination of linoleic and linolenic acids are deemed to be comprised within spirit and scope of the invention.

**Claims**

1. Use for the manufacture of a medicament for therapeutic application in treatment of Alzheimer's disease, related dementias and epilepsy of a composition which comprises a combination of (a) from 16.7 to 22.2% by weight of at least one compound selected from linolenic acid and derivatives thereof, calculated as the free acid, said derivatives of linolenic acid being both physiologically hydrolyzable and pharmacologically acceptable, and (b) balance to make 100% of said combination, of at least one compound selected from linoleic acid and derivatives thereof, calculated as the free acid, said derivatives of linoleic acid being both physiologically hydrolyzable and pharmacologically acceptable, provided that said composition does not include oily carrier or diluent selected from $C_{8-18}$ saturated fatty acids, oleic acid and derivatives of these acids.

2. Use according to claim 1 in which the composition comprises at least one pharmaceutically acceptable or orally ingestible substance selected from diluents, carriers and adjuvants.

3. Use according to claim 2, wherein said medicament is adapted for oral, parenteral or rectal administration.

4. Use according to claim 2 wherein said medicament is in depot form which will release ingredients (a) and (b) slowly in the body, over a preselected time period.

5. Use according to claim 2 wherein said medicament is in the form of a powder, tablet, capsule, solution, concentrate, syrup, suspension, gel or dispersion.

6. Use according to claim 2, wherein said diluent or carrier comprises one or more of a sugar-based confectionery, a manufactured cereal, a fruit or vegetable product, a beverage or beverage concentrate, a ground meat product or a vegetable analogue thereof, and optionally at least one additional ingredient selected from the water-soluble vitamins thiamine, riboflavin, niacin, pyridoxine, pantothenic acid, biotin, folic acid, cobalamin and ascorbic acid, the oil-soluble vitamins retinol, calciferol, tocopherol and menadione, in combined form the elements sodium, potassium, calcium, phosphorus, magnesium, chlorine and sulfur, iron, copper, iodine, manganese, cobalt, zinc, molybdenum, fluorine, selenium and chromium, unsaturated fatty acids which are known to be metabolized in the body to prostaglandins, and physiologically compatible derivatives (such as salts, esters and amides) of said fatty acids, and acceptable antioxidants, dispersing and suspending agents, and water.

7. Use according to any one of claims 1 to 6 wherein the said derivatives of linolenic and/or linoleic acids are salts, esters, or amides thereof.

8. Use according to any one of claims 1 to 7 wherein the said combination contains of about 19.0% of (a) and the balance to 100% of (b).

**Patentansprüche**

1. Verwendung einer Zusammensetzung zur Herstellung eines Arzneimittels zur therapeutischen Anwendung bei der Behandlung der Alzheimer' Krankheit, verwandter Arten von Dementia und Epilepsie, wobei die Zusammensetzung die Kombination umfaßt von (a) 16.7 bis 22.2 Gewichtsprozent von mindestens einer Verbindung ausgewählt aus Linolensäure und Derivaten davon, berechnet als freie Säure, wobei diese Derivate der Linolensäure physiologisch hydrolysierbar und pharmakologisch annehmbar sind, und (b) als Rest auf 100 % der Kombination, mindestens einer Verbindung ausgewählt aus Linolsäure und Derivaten davon, berechnet als freie Säure, wobei diese Derivate der Linolsäure physiologisch hydrolysierbar und pharmazeutisch annehmbar sind, mit der Maßnahme, daß die Zusammensetzung keinen öligen Träger oder Verdünner enthält, der ausgewählt ist aus C8-C18-gesättigten Fettsäuren, Ölsäure und Derivaten dieser Säuren.

2. Verwendung nach Anspruch 1, dadurch gekennzeichnet, daß die Zusammensetzung mindestens eine pharmazeutisch annehmbare oder oral aufnehmbare Substanz umfaßt ausgewählt aus Verdünnern, Trägern und Adjuvantien.

3. Verwendung nach Anspruch 2, dadurch gekennzeichnet, daß das Arzneimittel zur oralen, parenteralen oder rektalen Verabreichung geeignet ist.

4. Verwendung noch Anspruch 2, dadurch gekennzeichnet, daß das Arzneimittel in Depotform vorliegt, die die Bestandteile (a) und (b) im Körper langsam während einer vorgegebenen Zeitspanne freisetzt.

5. Verwendung nach Anspruch 2, dadurch gekennzeichnet, daß das Arzneimittel in Form eines Pulvers, von Tabletten, Kapseln, einer Lösung, eines Konzentrats, eines Sirups, einer Suspension, eines Gels oder einer Dispersion vorliegt.

6. Verwendung nach Anspruch 2, dadurch gekennzeichnet, daß der Verdünner oder Träger ein oder mehrere Konfekts auf Zuckerbasis, ein Getreideprodukt, ein Frucht- oder Gemüseprodukt, ein Getränk oder eine Getränkekonzentrat, ein gemahlenes Fleischprodukt oder ein Gemüseanaloges davon umfaßt, und gegebenenfalls mindestens einen zusätzlichen Bestandteil, ausgewählt aus den wasserlöslichen Vitaminen Thiamin, Riboflavin, Niacin, Pyridoxin, Pantothensäure, Biotin, Folsäure, Cobalamin und Ascorbinsäure, den öllöslichen Vitaminen Retinol, Calciferol, Tocopherol und Menadion, der kombinierten Form der Elemente Natrium, Kalium, Calcium, Phosphor, Magnesium, Chlor und Schwefel, Eisen, Kupfer, Iod, Mangan, Cobalt, Zink, Molybdän, Fluor, Selen und Chrom, den ungesättigten Fettsäuren, von denen es bekannt ist, daß sie im Körper zu Prostaglandinen metabolisiert werden, und physiologisch annehmbaren Derivaten (wie z.B. Salze, Ester und Amide) dieser Fettsäuren, und annehmbaren Antioxidantien, Dispergiermitteln und Suspendiermitteln, und Wasser.

7. Verwendung nach einem der Ansprüche 1 bis 6, dadurch gekennzeichnet, daß die Derivate der Linolensäure und/oder Linolsäure Salze, Ester oder Amide davon sind.

8. Verwendung nach einem der Ansprüche 1 bis 7, dadurch gekennzeichnet, daß die Kombination ca. 19.0 % von (a) und als Rest auf 100 % (b) enthält.

**Revendications**

1. Utilisation, pour la fabrication d'un médicament à des fins thérapeutiques, dans le traitement de la maladie d'Alzheimer, de démences apparentées et de l'épilepsie, d'une composition comprenant une combinaison contenant (a) de 16,7 à 22,2 % en poids, calculé d'après la forme acide libre, d'au moins un composé choisi parmi l'acide linolénique et des dérivés de celui-ci, lesdits dérivés de l'acide linolénique étant à la fois hydrolysables dans des conditions physiologiques et pharmaceutiquement acceptables, et (b) le complément pour amener ladite composition à 100 %, calculé d'après la forme acide libre, d'au moins un composé choisi parmi l'acide linoléique et des dérivés de celui-ci, lesdits dérivés de l'acide linoléique étant à la fois hydrolysables dans des conditions physiologiques et pharmaceutiquement acceptables, sous réserve que ladite composition ne comprenne pas de véhicule à base d'huile ou de diluant choisi parmi les acides saturés en $C_{8-18}$, l'acide oléique et des dérivés de ces acides.

2. Utilisation, selon la revendication 1, dans laquelle la composition comprend au moins une substance pharmaceutiquement acceptable ou convenant à une administration orale, choisie parmi les diluants, véhicules et adjuvants.

3. Utilisation selon la revendication 2, dans laquelle ledit médicament convient à une administration orale, parentérale ou rectale.

4. Utilisation selon la revendication 2, dans laquelle ledit médicament est sous une forme de dépôt qui libère les constituants (a) et (b) lentement dans le corps, au cours d'une période pré-définie.

5. Utilisation selon la revendication 2, dans laquelle le médicament est sous forme de poudre, de comprimé, de capsule, de solution, de concentré, de sirop, de suspension, de gel ou de dispersion.

**6.** Utilisation selon la revendication 2, dans laquelle ledit diluant ou le véhicule comprend une ou plusieurs confiseries à base de sucre, une préparation de céréale, un produit à base de fruit ou de légume, une boisson ou une boisson concentrée, un produit à base de viande hachée ou un analogue à base de légume de celui-ci et, éventuellement, au moins un constituant supplémentaire choisi parmi les vitamines hydrosolubles, la thiamine, la riboflavine, la niacine, la pyridoxine, l'acide pantothénique, la biotine, l'acide folique, la cobalamine et l'acide ascorbique, les vitamines liposolubles, le rétinol, le calciférol, le tocophérol et la ménadione, sous forme combinée les éléments sodium, potassium, calcium, phosphore, magnésium, chlore et soufre, fer, cuivre, iode, manganèse, cobalt, zinc, molybdène, fluor, sélénium et chrome, des acides gras insaturés qui sont connus pour être métabolisés dans le corps en prostaglandines, et des dérivés physiologiquement compatibles (tel que des sels, des esters et des amides) desdits acides gras, et des antioxydants acceptables, des agents de suspension et de dispersion, et de l'eau.

**7.** Utilisation selon l'une quelconque des revendications 1 à 6 dans lesquelles lesdits dérivés des acides linoléniques et/ou linoléiques sont des sels, des esters ou des amides de ceux-ci.

**8.** Utilisation selon l'une quelconque des revendications 1 à 7, dans lesquelles ladite combinaison contient environ 19,0 % de (a) et le complément à 100 % de (b).